# EUROPEAN PATENT APPLICATION

(11) **EP 4 814 811 A1**
(43) Date of publication of application: **30.09.2026**
(21) Application number: 23962247.5
(22) Date of filing: 21.12.2023
(51) Int. Cl.: G06N 20/00

(54) **INFORMATION PROCESSING SYSTEM, INFORMATION PROCESSING METHOD, INFORMATION PROCESSING PROGRAM, AND MOLECULAR COMPOUND PRODUCTION METHOD**

(71) Applicant: CHUGAI SEIYAKU KABUSHIKI KAISHA, Tokyo 115-8543 (JP)
(72) Inventor: YOSHIMURA Dai, Yokohama City, Kanagawa 244-8602 (JP); SHIRAI Kazuhide, Yokohama City, Kanagawa 244-8602 (JP); TERAMOTO Reiji, Yokohama City, Kanagawa 244-8602 (JP); TAKIZAWA Shuuki, Yokohama City, Kanagawa 244-8602 (JP)
(74) Representative: Vossius & Partner Patentanwälte Rechtsanwälte mbB
(86) International application number: PCT/JP2023/045988
(87) International publication number: WO 2025/134327

(57) **Abstract**

An information processing system of the present disclosure comprises at least one processor. The at least one processor acquires, for each of a plurality of first molecules, training data representing a plurality of feature values related to the first molecule, acquires, for each of a plurality of second molecules, test data representing a plurality of feature values related to the second molecule, calculates, for each of a plurality of feature values, an index based on a first probability distribution which is a probability distribution in the training data and a second probability distribution which is a probability distribution in the test data, and selects, on the basis of the index of each of a plurality of feature values, one or more of the plurality of feature values as one or more input feature values to be used as input parameters of a prediction model for predicting a characteristic value of a molecule on the basis of machine learning.

## Description

### [Technical Field]

An aspect of the present disclosure relates to an information processing system, an information processing method, an information processing program, and a method for producing a molecular compound.

### [Background Art]

Patent Literature 1 discloses a method for identifying an amino acid sequence of an antibody having affinity for an antigen. The method comprises the steps of asking a machine learning engine about a proposed amino acid sequence of an antibody having high affinity for an antigen, and acquiring the proposed amino acid sequence from the machine learning engine.

Non Patent Literature 1 discloses a technique for selecting feature values in training data and test data. In this technique, when the score of classification between training data and test data by an adversarial classifier is higher than a predetermined threshold, feature values that are of high importance in the classification are excluded, and the adversarial classifier is trained again. When the score becomes lower than the threshold, a prediction model is trained using the remaining feature values.

### [Citation List]

### [Patent Literature]

[Patent Literature 1] International Publication No. WO 2018/132752

### [Non Patent Literature]

[Non Patent Literature 1] Pan, Jing, et al. "Adversarial validation approach to concept drift problem in user targeting automation systems at uber." arXiv: 2004.03045 (2020).

### [Summary of Invention]

### [Technical Problem]

It is desirable to improve the accuracy of a machine learning model for predicting characteristics of a molecule.

### [Solution to Problem]

An information processing system according to an aspect of the present disclosure comprises at least one processor. The at least one processor acquires, for each of a plurality of first molecules, training data representing a plurality of feature values related to the first molecules, acquires, for each of a plurality of second molecules, test data representing a plurality of feature values related to the second molecule, calculates, for each of a plurality of feature values, an index based on a first probability distribution which is a probability distribution in the training data and a second probability distribution which is a probability distribution in the test data, and selects, on the basis of the index of each of a plurality of feature values, one or more of the plurality of feature values as one or more input feature values to be used as input parameters of a prediction model for predicting a characteristic value of a molecule on the basis of machine learning.

An information processing method according to an aspect of the present disclosure is executed by an information processing system comprising at least one processor. The information processing method comprises the steps of: acquiring, for each of a plurality of first molecules, training data representing a plurality of feature values related to the first molecule; acquiring, for each of a plurality of second molecules, test data representing a plurality of feature values related to the second molecule; calculating, for each of a plurality of feature values, an index based on a first probability distribution which is a probability distribution in the training data and a second probability distribution which is a probability distribution in the test data; and selecting, on the basis of the index of each of a plurality of feature values, one or more of the plurality of feature values as one or more input feature values to be used as input parameters of a prediction model for predicting a characteristic value of a molecule on the basis of machine learning.

An information processing program according to an aspect of the present disclosure allows a computer to execute the steps of: acquiring, for each of a plurality of first molecules, training data representing a plurality of feature values related to the first molecule; acquiring, for each of a plurality of second molecules, test data representing a plurality of feature values related to the second molecule; calculating, for each of a plurality of feature values, an index based on a first probability distribution which is a probability distribution in the training data and a second probability distribution which is a probability distribution in the test data; and selecting, on the basis of the index of each of a plurality of feature values, one or more of the plurality of feature values as one or more input feature values to be used as input parameters of a prediction model for predicting a characteristic value of a molecule on the basis of machine learning.

In these aspects, feature values to be used as input parameters of a prediction model based on machine learning are selected as input feature values on the basis of the tendency of each feature value in both training data and test data. By selecting feature values as described above, the accuracy of a machine learning model (prediction model) for predicting characteristics of a molecule can be improved.

### [Advantageous Effect of Invention]

According to an aspect of the present disclosure, the accuracy of a machine learning model for predicting characteristics of a molecule can be improved.

### [Brief Description of Drawings]

[Figure 1] Figure 1 shows a diagram for illustrating selection of an input feature value.
[Figure 2] Figure 2 shows a diagram illustrating an example of a functional configuration of an information processing system.
[Figure 3] Figure 3 shows a diagram illustrating an example of a hardware configuration of a computer that functions as an information processing system.
[Figure 4] Figure 4 shows a diagram illustrating an example of training data.
[Figure 5] Figure 5 shows a flowchart illustrating an example of a process executed by an information processing system.
[Figure 6] Figure 6 shows a flowchart illustrating an example of a process for selecting an input feature value.
[Figure 7] Figure 7 shows a diagram for illustrating cross-validation.
[Figure 8] Figure 8 shows a flowchart illustrating an example of cross-validation.
[Figure 9] Figure 9 shows a flowchart illustrating another example of a process for selecting an input feature value.
[Figure 10] Figure 10 shows a diagram illustrating a first example of validation.
[Figure 11] Figure 11 shows a diagram illustrating a second example of validation.

### [Description of Embodiments]

Hereinafter, various examples of the present disclosure will be described in detail with reference to the accompanying drawings. In description of the drawings, identical or equivalent elements are provided with the same reference signs, and redundant description thereof is omitted.

### [Outline of system]

An information processing system according to the present disclosure is a computer system that selects one or more feature values of a molecule which are used as input parameters of a prediction model for predicting a characteristic value of the molecule on the basis of machine learning. In the present disclosure, the feature value used as an input parameter is referred to as an "input feature value". The information processing system excludes some feature values from a plurality of feature values which are candidates for input feature values, followed by selection of the remaining one or more feature values, as one or more input feature values. The number of feature values is also referred to as the number of dimensions. The process for selecting one or more input feature values from a plurality of feature values can also be said to be a process for reducing the number of dimensions of the feature value.

In an example, the information processing system executes machine learning using the selected one or more input feature values, thereby generating a relevant prediction model. This process corresponds to a learning phase of machine learning. In an example, the information processing system inputs one or more input feature values of a molecule whose characteristic value is unknown to the generated prediction model, thereby predicting the characteristic value of the molecule. In the present disclosure, the molecule whose characteristic value is predicted by the information processing system is also referred to as a "molecule of interest". The information processing system may execute screening in which a characteristic value of each of a plurality of molecules of interest is predicted, and at least one molecule of interest is selected from the plurality of molecules of interest on the basis of the predicted characteristic values. The prediction of characteristic values corresponds to a prediction phase or an operation phase of machine learning. In the learning phase and the prediction phase, the information processing system generates and uses a prediction model with the selected one or more input feature values.

Machine learning is a technique for autonomously finding out a law or a rule by iteratively learning on the basis of given information. The prediction model generated by machine learning is a machine learning model constructed using an algorithm and a data structure, and is also referred to as a learned model. In an example, the prediction model is constructed by a neural network such as a convolutional neural network (CNN).

In the present disclosure, the feature value is a numerical value that represents a quantified feature of a molecule. In an example, a plurality of feature values are set for each molecule. Each feature value may represent a feature of a relationship among a plurality of constituent units forming a molecule, a sequence of the plurality of constituent units, or a feature of a specific constituent unit.

In the present disclosure, the characteristic value is a numerical value that represents a quantified characteristic of a molecule. For example, the characteristic value can represent various characteristics related to binding ability, affinity, pharmacological activity, physical properties, kinetics or safety of a molecule.

The information processing system selects an input feature value. A prediction model is generated by machine learning using training data on a molecule. A predicted value of a characteristic of a molecule shown by test data is calculated on the basis of the prediction model. In the present disclosure, the prediction model generated is also referred to as a "characteristic prediction model".

In the present disclosure, each of molecules shown by training data is referred to as a "first molecule". Each of molecules shown by test data is referred to as a "second molecule". The first molecule and the second molecule may be identical or different in modality. As an example, the first molecule is an antibody, and the second molecule is an antibody. As another example, the first molecule is a cyclic peptide, and the second molecule is a cyclic peptide. As still another example, when affinity is predicted using energy of interaction between a ligand and a protein as a feature value, the first molecule may be a small molecule, with the second molecule being a cyclic peptide.

A group of first molecules comprises a plurality of first molecules. The training data includes, for each of a plurality of first molecules, a plurality of feature values related to the first molecule. A group of second molecules comprises a plurality of second molecules. The test data includes, for each of a plurality of second molecules, a plurality of feature values related to the second molecule. A plurality of first molecules in the group of first molecules may be identical or different in modality. A plurality of second molecules in the group of second molecules may be identical or different in modality.

As an example, when the molecule is an antibody, training data representing an antibody sequence is used. In the case of a molecule having sequence information as described above, the group of first molecules may be rephrased as a group of sequences for training, and the group of second molecules may be rephrased as a group of sequences for prediction.

The training data includes, for each of a plurality of first molecules, a plurality of feature values of the first molecule, and at least one characteristic value of the first molecule. The test data includes, for each of a plurality of second molecules, a plurality of feature values of the second molecule. If there are a relatively large number of feature values which vary in tendency between training data and test data, the accuracy of the prediction model deteriorates. In the present disclosure, the information processing system selects one or more input feature values such that feature values which vary in tendency between training data and test data do not affect machine learning and the prediction model. In an example, the information processing system selects feature values which are relatively small in difference in tendency between training data and test data. In an example, the information processing system excludes feature values which significantly vary in tendency between training data and test data.

In an example, the information processing system calculates, for each of a plurality of feature values, a distribution distance, which is a distance between a first probability distribution which is a probability distribution in training data and a second probability distribution which is a probability distribution in test data, as a selection index. The probability distribution of a feature value is a distribution showing the probabilities of respective values assumed by the feature value. Since the probability distribution represents a tendency of a feature value, feature values which are relatively large in difference between the first probability distribution and the second probability distribution can be said to vary in tendency between training data and test data. The selection index is an index for selecting an input feature value.

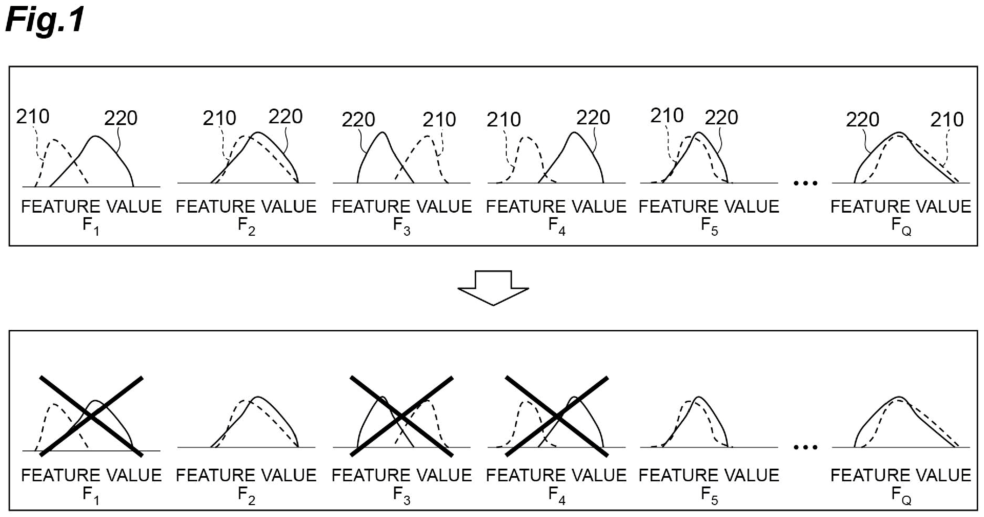

Figure 1 shows a diagram for illustrating selection of an input feature value. In this example, the information processing system calculates, for each of Q feature values, a distribution distance, which is a distance between a first probability distribution 210 in training data and a second probability distribution 220 in test data, as a selection index. The information processing system excludes feature values which are relatively large in difference between the first probability distribution 210 and the second probability distribution 220 (for example, feature values F₁, F₃, F₄), followed by selection of the remaining feature values (for example, feature values F₂, F₅, F_{Q}) as input feature values. Each of the input feature values selected is a feature value which is relatively small in difference between the first probability distribution 210 and the second probability distribution 220, and relatively consistent in tendency. Therefore, a prediction model generated by machine learning based on the input feature value is capable of predicting the characteristic value of a molecule of interest.

### [Configuration of system]

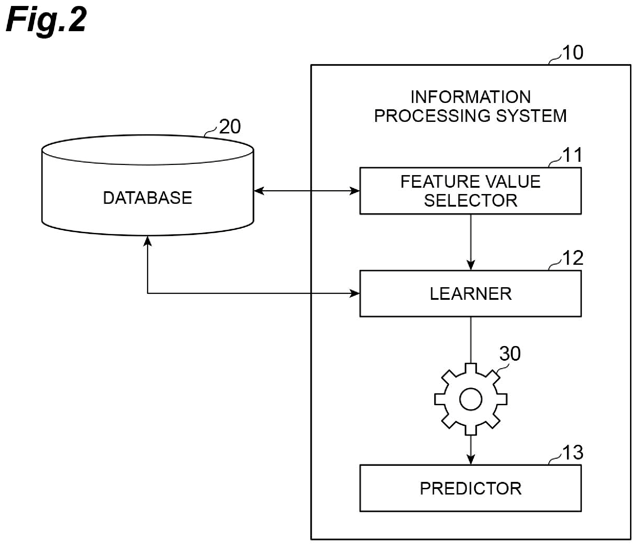

Figure 2 shows a diagram illustrating a functional configuration of an information processing system 10 according to an example. In this example, the information processing system 10 accesses a database 20 for storing training data used for machine learning. The database 20 may be provided in a computer system different from the information processing system 10, or may be a constituent element of the information processing system 10. In an example, the information processing system 10 accesses the database 20 through a communication network such as internet or intranet.

The information processing system 10 comprises a feature value selector 11, a learner 12, and a predictor 13. The feature value selector 11 is a functional module that selects one or more input feature values from a plurality of feature values. The learner 12 is a functional module that generates a prediction model 30 by machine learning based on the selected one or more input feature values. The predictor 13 is a functional module that executes prediction related to a molecule of interest using the generated prediction model 30.

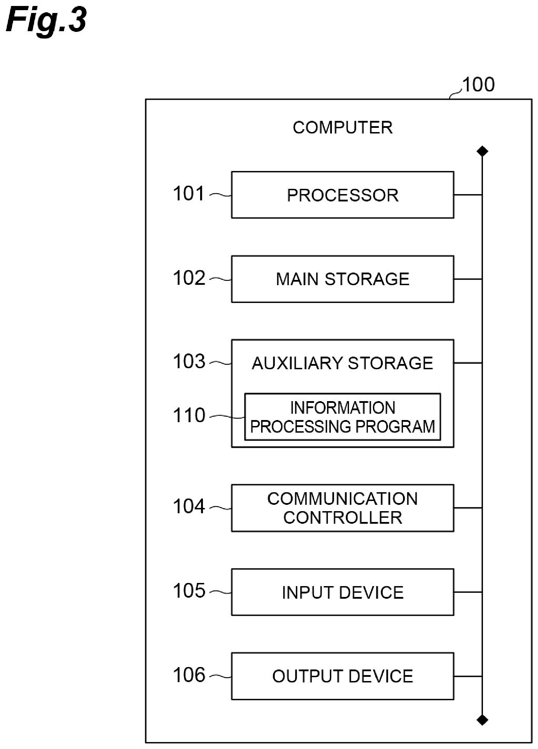

Figure 3 shows a diagram illustrating an example of a hardware configuration of a computer 100 that functions as the information processing system 10. For example, the computer 100 comprises a processor 101, a main storage 102, an auxiliary storage 103, a communication controller 104, an input device 105, and an output device 106. The processor 101 executes an operating system and application programs. The main storage 102 comprises, for example, ROM and RAM. The auxiliary storage 103 comprises a hard disk or a flash memory, and typically stores a larger amount of data than the main storage 102. The communication controller 104 comprises, for example, a network card or a wireless communication module. The input device 105 comprises, for example, a keyboard, a mouse, and a touch panel. The output device 106 comprises, for example, a monitor and a speaker.

The functional modules of the information processing system 10 are implemented by an information processing program 110 which is stored in the auxiliary storage 103 in advance. Specifically, the functional modules are implemented by allowing the information processing program 110 to be read on the processor 101 or the main storage 102, with the processor 101 executing the information processing program 110. The processor 101 causes the communication controller 104, the input device 105 or the output device 106 to operate in accordance with the information processing program 110, and reads and writes data from and to the main storage 102 or the auxiliary storage 103. Data, a prediction model, or a database necessary for the processing may be stored in the main storage 102 or the auxiliary storage 103.

The information processing program 110 may be recorded in a non-transitory computer-readable storage medium such as CD-ROM, DVD-ROM or a semiconductor memory, and provided. Alternatively, the information processing program 110 may be provided via a communication network as data signals superimposed on a carrier wave.

The information processing system 10 may comprise one computer 100, or may comprise a plurality of computers 100. When a plurality of computers 100 are used, the computers 100 are connected via a communication network such as internet or intranet to construct logically one information processing system 10.

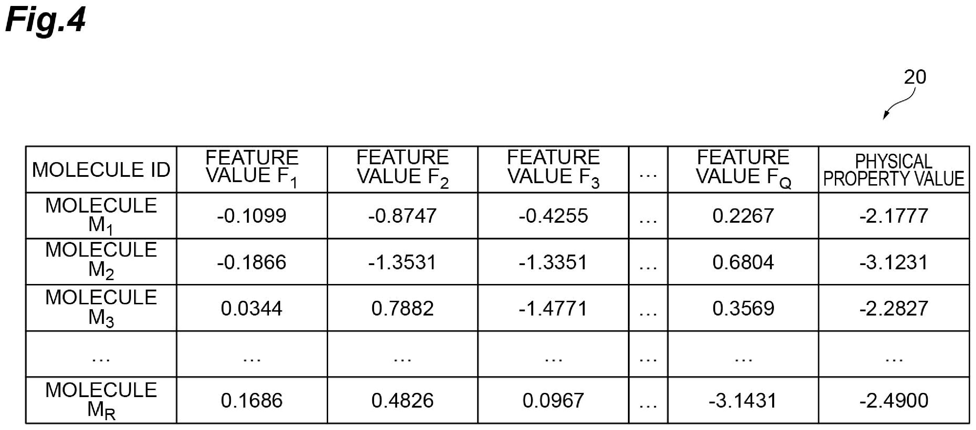

Figure 4 shows a diagram illustrating an example of training data that is stored in the database 20. The training data comprises a plurality of data records corresponding to a plurality of molecules. In an example, each data record comprises, as data item, a molecule ID which is an identifier that uniquely identifies each molecule, a plurality of feature values of the molecule, and a characteristic value of the molecule. When the molecule is a protein, a plurality of feature values used as training data may be acquired by, for example, a tasks assessing protein embeddings method (TAPE). In the example of Figure 4, the database 20 stores R data records each representing Q feature values (that is, a feature value of Q dimensions). The number of dimensions Q of the feature value may be less than 10, or may be in the order of tens, hundreds, thousands or tens of thousands. The information processing system 10 excludes some feature values from Q feature values, followed by selection of the remaining one or more feature values, as one or more input feature values. In the example 4, a physical property value is shown as a characteristic value of a molecule, but the characteristic value is not limited to a physical property value.

### [Operation of system]

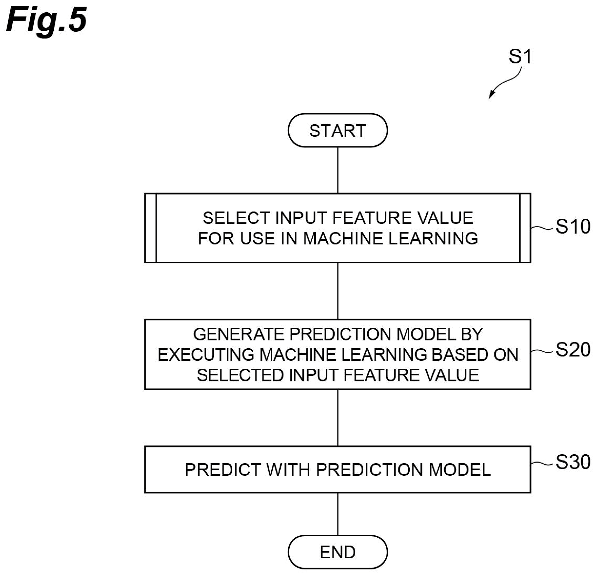

Figure 5 shows a flowchart illustrating an example of a process executed by the information processing system 10, as a process flow S1. The process flow S1 is an example of an information processing method according to the present disclosure. In step S 10, the feature value selector 11 selects one or more input feature values for use in machine learning from a plurality of feature values represented by training data. In an example, the feature value selector 11 calculates a plurality of evaluation indices with some feature values being selected or excluded, in which the evaluation indices are calculated while changing the feature values to be selected or excluded. The feature value selector 11 selects, as input feature values, a set of feature values when the evaluation index meets a predetermined condition. In step S20, the learner 12 generates the prediction model 30 by executing machine learning based on the selected one or more input feature values. In step S30, the predictor 13 executes prediction using the prediction model 30. Step S10 corresponds to preprocessing of the learning phase, step S20 corresponds to the learning phase, and step S30 corresponds to the prediction phase. The one or more input feature values selected in step S10 are used in steps S20 and S30.

### (Selection of input feature value)

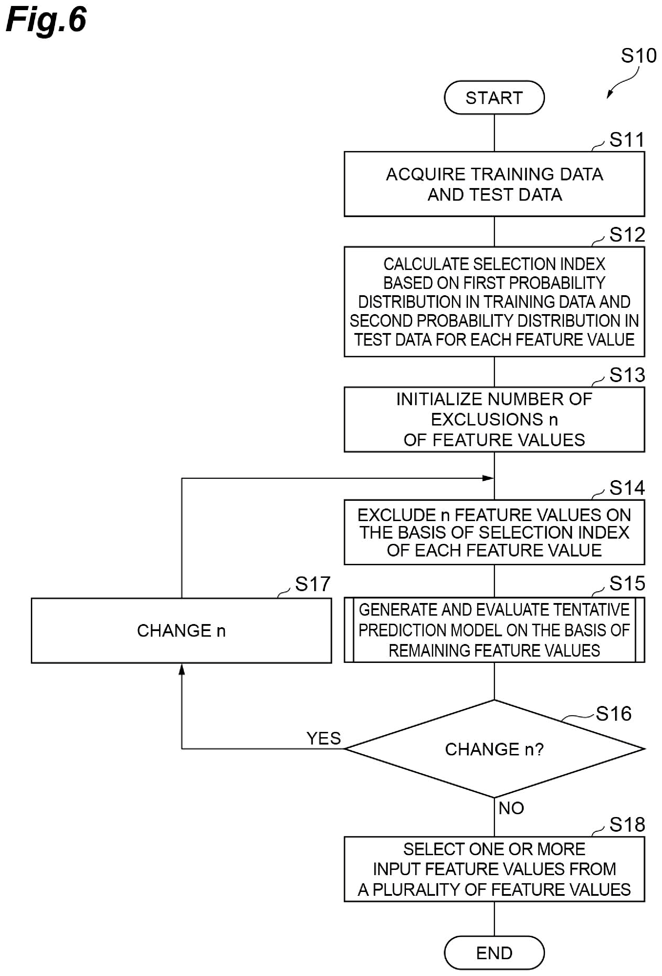

Figure 6 shows a flowchart illustrating an example of a process for selecting an input feature value, that is, an example of step S10, in detail.

In step S11, the feature value selector 11 acquires training data and test data from the database 20. The training data includes, for each of a plurality of first molecules, a plurality of feature values related to the first molecule. The test data includes, for each of a plurality of second molecules, a plurality of feature values related to the second molecule.

In step S12, the feature value selector 11 calculates, for each of a plurality of feature values, a selection index, which is an index for selecting an input feature value, on the basis of the first probability distribution in training data and the second probability distribution in test data. In an example, the feature value selector 11 calculates, for each feature value, a distribution distance, which is a distance between the first probability distribution and the second probability distribution, as a selection index. The distribution distance indicates a degree of the difference between the first probability distribution and the second probability distribution. The feature value selector 11 may use integral probability metrics (IPMs) as a distribution distance. As an example of IPMs, the feature value selector 11 may calculate a Wasserstein distance, a maximum mean discrepancy (MMD) or a Dudley metric as a distribution distance.

The feature value selector 11 may calculate the first probability distribution and the second probability distribution for calculating a selection index. A feature value is Fᵢ, the number of data records of training data is j, and the number of data records of test data is k. The feature value selector 11 may calculate, for the feature value Fᵢ, a probability distribution of j values as the first probability distribution, and a probability distribution of k values as the second probability distribution.

In step S13, the feature value selector 11 initializes the number of exclusions n of feature values. The number of exclusions n is the number of feature values excluded from a plurality of feature values on the basis of the selection index for each of a plurality of feature values. The number of exclusions n can also be said to be a numerical reduction of dimensions of the feature value. The feature value selector 11 changes the number of exclusions n in any of subsequent processes. When the number of exclusions n is incremented, the initial value of the numerical reduction n may be 0, or 1 or more. When the number of exclusions n is decremented, the initial value of the numerical reduction n may be {(total number of feature values) - 1}, or a smaller number.

In step S14, the feature value selector 11 excludes n feature values from a plurality of feature values on the basis of the selection index for each feature value. When a distribution distance such as a Wasserstein distance is used, the feature value selector 11 excludes n feature values in descending order of the distribution distance. That is, the feature value selector 11 excludes n feature values in which the distance between the first probability distribution and the second probability distribution is relatively large.

In step S15, the feature value selector 11 generates and evaluates a tentative prediction model on the basis of one or more feature values remaining after the exclusion process. In the present disclosure, the generation and evaluation of the tentative prediction model is also referred to as an "evaluation process". The tentative prediction model is not the prediction model 30 that is generated by the learner 12 and used by the predictor 13, but is a machine learning model (learned model) that is temporarily used for selecting one or more input feature values. The feature value selector 11 may use cross-validation or a hold-out method as a method for the evaluation process.

As an example of step S15, cross-validation will be described. In the cross-validation, the feature value selector 11 divides training data into a plurality of groups, selects one of the plurality of groups as validation data, and selects the remaining groups as narrowly defined training data. Each of the separated groups is also referred to as a "fold". A combination of validation data and narrowly defined training data is also referred to as a "split". The feature value selector 11 generates a tentative prediction model using narrowly defined training data, and evaluates the tentative prediction model using validation data. The feature value selector 11 executes generation and evaluation of the tentative prediction model while changing a group (fold) used as validation data, and calculates an evaluation index for the tentative prediction model for each split. The feature value selector 11 obtains, as an evaluation index of one cross-validation, the statistic of a plurality of evaluation indices obtained from a plurality of splits.

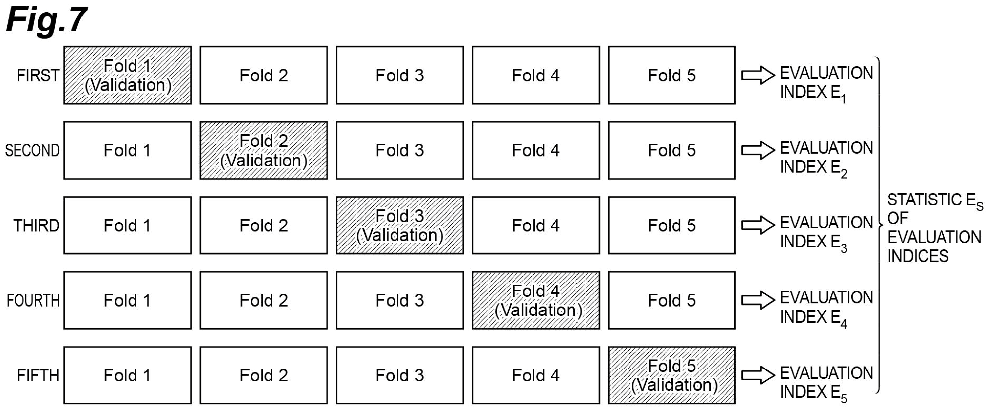

Figure 7 shows a diagram for illustrating cross-validation. In the example shown in this figure, the feature value selector 11 divides training data into five groups. In the first split, the feature value selector 11 selects "Fold 1" as validation data, and selects the remaining four groups as narrowly defined training data. The feature value selector 11 generates a tentative prediction model by machine learning using narrowly defined training data, and calculates an evaluation index E₁ of the tentative prediction model using validation data. The feature value selector 11 executes generation and evaluation of the tentative prediction model for second to fifth splits while changing the validation data, and calculates four evaluation indices E₂, E₃, E₄ and E₅ for the splits. The feature value selector 11 determines the statistic of the five evaluation indices E₁ to E₅ as a final evaluation index of one cross-validation. Examples of the statistic include a mean value and a median value, but the statistic is not limited thereto.

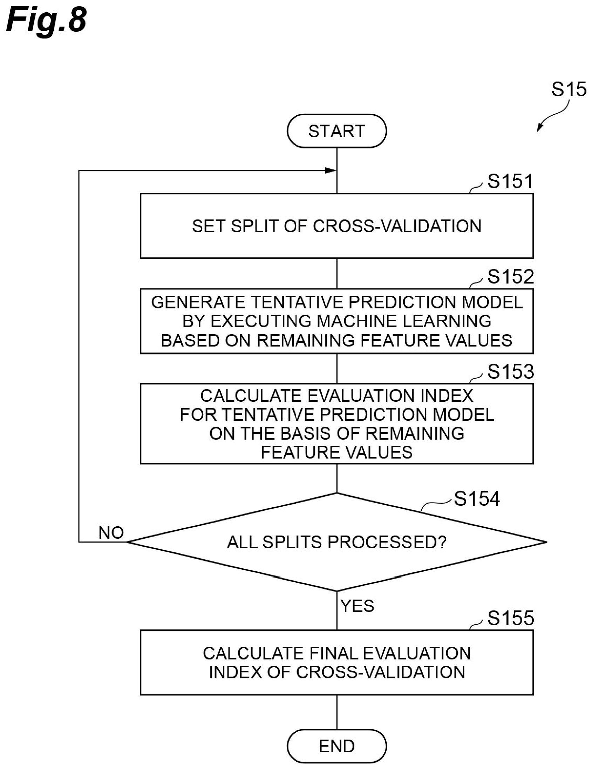

Figure 8 shows a flowchart illustrating an example of cross-validation. In step S151, the feature value selector 11 sets a split of cross-validation. The feature value selector 11 divides training data acquired from the database 20 into a plurality of groups, selects one of the plurality of groups as validation data, and selects the remaining groups as narrowly defined training data.

In step S152, the feature value selector 11 generates a tentative prediction model by executing machine learning based on one or more feature values remaining after the exclusion process. The feature value selector 11 generates the tentative prediction model by executing machine learning (supervised learning) using narrowly defined training data. The feature value selector 11 does not use the excluded n feature values, and inputs the remaining one or more feature values as input parameters (for example, input vectors) to the machine learning model. In an example, the feature value selector 11 updates a group of parameters in the machine learning model by executing backpropagation (a backpropagation method) based on an error between a predicted value calculated by the machine learning model and a correct solution (label). The learner 12 obtains the tentative prediction model by repeating the process until a given end condition is met. The end condition may be processing of all data records of narrowly defined training data.

In step S153, the feature value selector 11 calculates an evaluation index on the basis of the remaining one or more feature values. The evaluation index is a value that indicates how accurately the tentative prediction model can calculate a characteristic value. The feature value selector 11 does not use the excluded n feature values, and inputs the remaining one or more feature values as input parameters (for example, input vectors) to the tentative prediction model for each data record of validation data. The tentative prediction model calculates a characteristic value on the basis of the input parameters for each data record. Hereinafter, the characteristic value calculated by the tentative prediction model is also referred to as a predicted characteristic value.

The feature value selector 11 calculates an evaluation index for the tentative prediction model on the basis of the predicted characteristic value and a correct solution (label) for each data record of validation data. The correct solution (label) in the process is a characteristic value within the training data used as validation data. For example, the feature value selector 11 may calculate a mean square error between the prediction characteristic value and the correct solution, or a correlation coefficient that indicates a degree of correlation between the prediction characteristic value and the correct solution.

As shown in step S154, when there is a split that has not been executed (NO in step S154), the process returns to step S151. In step S151 that is repeated, the feature value selector 11 changes the group (fold) to be used as validation data, and sets a next split. In step S152 that is repeated, the feature value selector 11 generates the tentative prediction model by executing, on the basis of the remaining one or more feature values, machine learning (supervised learning) using narrowly defined training data. In step S153 that is repeated, the feature value selector 11 calculates an evaluation index for the tentative prediction model on the basis of the remaining one or more feature values.

When all splits are processed (YES in step S154), the process proceeds to step S155. In step S155, the feature value selector 11 calculates a statistic of the evaluation indices of each split as a final evaluation index of cross-validation. For example, the feature value selector 11 calculates an average value of mean square errors or an average value of correlation coefficients as a final evaluation index.

Again, with reference to Figure 6, in step S16, the feature value selector 11 determines whether the number of exclusions n is changed. The change of the number of exclusions n may be an increment or a decrement.

In an example, the feature value selector 11 determines whether the number of exclusions n is incremented, on the basis of the number of feature values excluded by an exclusion process, or the number of feature values which have not been excluded by the exclusion process. For example, the feature value selector 11 determines that n is incremented when the effective number of dimensions which is the number of feature values remaining after the exclusion process is equal to or greater than a predetermined threshold, and that n is not incremented when the effective number of dimensions is smaller than the threshold. Alternatively, the feature value selector 11 determines that n is incremented when the effective number of dimensions is greater than a predetermined threshold, and that n is not incremented when the effective number of dimensions is equal to or smaller than the threshold. In another example, the feature value selector 11 determines that n is incremented when the number of feature values excluded by the exclusion process is equal to or smaller than a predetermined threshold, and that n is not incremented when the number the feature values is greater than the threshold. Alternatively, the feature value selector 11 determines that n is incremented when the number of feature values excluded by the exclusion process is smaller than a predetermined threshold, and that n is not incremented when the number the feature values is equal to or greater than the threshold.

In an example, the feature value selector 11 determines whether the number of exclusions n is decremented, on the basis of the number of feature values excluded by an exclusion process, or the number of feature values which have not been excluded by the exclusion process. For example, the feature value selector 11 determines that n is decremented when the effective number of dimensions which is the number of feature values remaining after the exclusion process is equal to or smaller than a predetermined threshold, and that n is not decremented when the effective number of dimensions is greater than the threshold. Alternatively, the feature value selector 11 determines that n is decremented when the effective number of dimensions is smaller than a predetermined threshold, and that n is not decremented when the effective number of dimensions is equal to or greater than the threshold. In another aspect, the feature value selector 11 determines that n is decremented when the number of feature values excluded by the exclusion process is equal to or greater than a predetermined threshold, and that n is not decremented when the number the feature values is smaller than the threshold. In another example, the feature value selector 11 determines that n is decremented when the number of feature values excluded by the exclusion process is greater than a predetermined threshold, and that n is not decremented when the number the feature values is equal to or smaller than the threshold.

When the number of exclusions n is changed (YES in step S16), the process proceeds to step S17. In step S17, the feature value selector 11 changes the number of exclusions n by a predetermined number. The change is an increment or a decrement. The predetermined number is any positive integer. For example, the feature value selector 11 increases the number of exclusions n by 1, 2, 3, 4, 5, 6, 7, 8, 9 or 10. As another example, the feature value selector 11 increases the number of feature values excluded by a number equal to the initial value of n. Alternatively, the feature value selector 11 reduces the number of exclusions n by 1, 2, 3, 4, 5, 6, 7, 8, 9 or 10. After step S17, the process returns to step S14. In step S14 that is repeated, the feature value selector 11 excludes n feature values from a plurality of feature values on the basis of the selection index for each feature value. In step S15 that is repeated, the feature value selector 11 generates and evaluates a tentative prediction model on the basis of the remaining one or more feature values. The feature value selector 11 gradually increases or reduces the number of exclusions n to calculate an evaluation index for the tentative prediction model for each number of exclusions n.

When the number of exclusions n is not changed (NO in step S16), the process proceeds to step S18. In step S18, the feature value selector 11 selects one or more input feature values from a plurality of feature values. In an example, the feature value selector 11 determines the final number of exclusions n_{FINAL} on the basis of a final evaluation index obtained for each number of exclusions. The feature value selector 11 may determine, as the number of exclusions n_{FINAL}, the number of exclusions at which the best final evaluation index is obtained. For example, the feature value selector 11 may determine, as the number of exclusions n_{FINAL}, the number of exclusions at which the highest final evaluation index or the lowest final evaluation index is obtained. As an example thereof, when the statistic of the mean square error is an evaluation index, the feature value selector 11 may determine, as the number of exclusions n_{FINAL}, the number of exclusions at which the lowest final evaluation index is obtained. When the statistic of the correlation coefficient is an evaluation index, the feature value selector 11 may determine, as the number of exclusions n_{FINAL}, the number of exclusions at which the highest final evaluation index is obtained. That is, the feature value selector 11 may determine, as the number of exclusions n_{FINAL}, the number of exclusions n with the smallest statistic of the mean square error, or the number of exclusions with the highest statistic of the correlation coefficient. Alternatively, the feature value selector 11 may determine, as the number of exclusions n_{FINAL}, one of one or more numbers of exclusions at which the final evaluation index is higher as compared to a case where a plurality of feature values are all used. Alternatively, the feature value selector 11 may determine, as the number of exclusions n_{FINAL}, one of one or more numbers of exclusions at which a decrease in final evaluation index as compared to a case where a plurality of feature values are all used is smaller than a predetermined threshold. The feature value selector 11 excludes n_{FINAL} feature values from a plurality of feature values on the basis of the selection index for each feature value. When a distribution distance such as a Wasserstein distance is used, the feature value selector 11 excludes n_{FINAL} feature values in descending order of the distribution distance, and selects the remaining one or more feature values as one or more input feature values. In this case, the feature value selector 11 selects, as one or more input feature values, one or more feature values in which the distribution distance is smaller than a predetermined reference determined on the basis of the final evaluation index. The predetermined reference may be determined by a user or an administrator of the information processing system 10, or may be automatically determined by any technique such as machine learning.

As described above, the feature value selector 11 excludes a part of a plurality of feature values on the basis of the selection index for each of a plurality of feature values. The feature value selector 11 selects, as one or more input feature values, the one or more feature values remaining after the exclusion process. In an example, the feature value selector 11 calculates, for each of a plurality of feature values, a distribution distance which is a distance between the first probability distribution and the second probability distribution, followed by selection of one or more input feature values from a plurality of feature values on the basis of the distribution distance for each of a plurality of feature values. In an example, the feature value selector 11 calculates, for each of a plurality of feature values, a distribution distance which is a distance between the first probability distribution and the second probability distribution, followed by selection of feature values in which the distribution distance for each of a plurality of feature values is relatively small, as input feature values.

As described above, in an example, the feature value selector 11 repeats an evaluation process in which generation of a tentative prediction model and calculation of an evaluation index for the tentative prediction model are executed on the basis of the remaining one or more feature values while changing the number of feature values excluded from a plurality of feature values on the basis of a selection index for each of a plurality of feature values (steps S14 to S17 are repeated). The feature value selector 11 can execute the evaluation process by cross-validation. The feature value selector 11 determines the number of exclusions on the basis of a plurality of evaluation indices obtained by repeating the evaluation process (step S18). The feature value selector 11 excludes feature values by the number of exclusions n_{FINAL} from a plurality of feature values on the basis of the selection index for each of a plurality of feature values to select one or more input feature values (step S18).

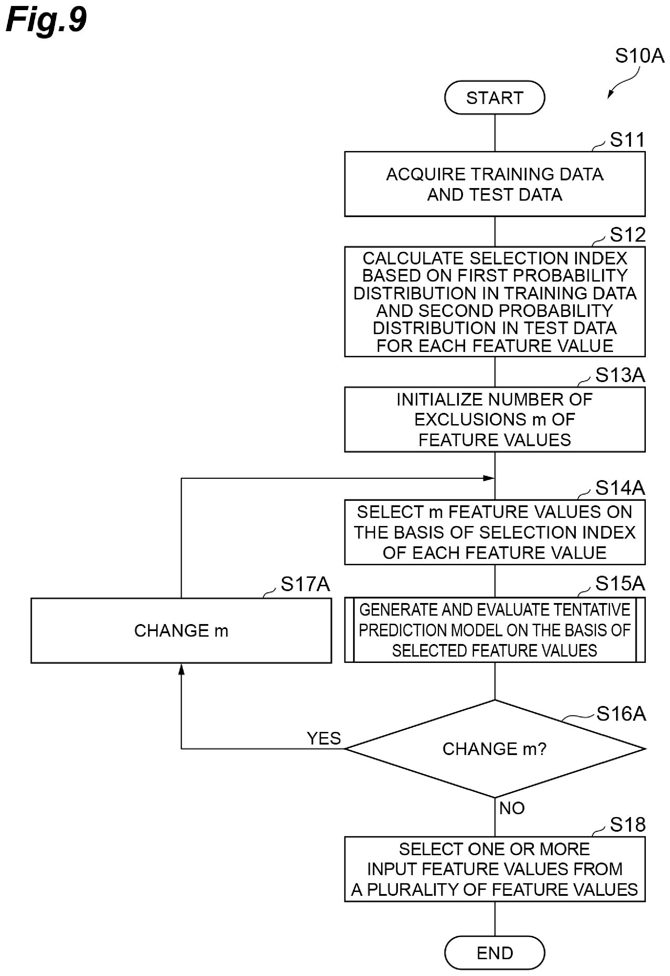

With reference to Figure 9, another example of a process for selecting input feature values will be described. Figure 9 shows a flowchart illustrating the example as step S10A in detail. Step S10A can also be said to be a modification of step S10. Step S10A is different from step S10 in that generation and evaluation of a tentative prediction model are repeated while instead of the number of exclusions n of feature values, the number of selections m of feature values is changed. Hereinafter, the difference will be mainly described.

As in step S10, the feature value selector 11 executes the processes of steps S11 and S12.

In step S13A, the feature value selector 11 initializes the number of selections m of feature values. The number of selections m is the number of feature values selected from a plurality of feature values on the basis of the selection index for each of a plurality of feature values. When the number of selections m is incremented, the initial value of the number of selections m may be 1, or 2 or more. When the number of selections m is decremented, the initial value of the number of selections m may be the total number of feature values, or a smaller number.

In step S14A, the feature value selector 11 selects m feature values from a plurality of feature values on the basis of the selection index for each feature value. When a distribution distance such as a Wasserstein distance is used, the feature value selector 11 selects m feature values in ascending order of the distribution distance. That is, the feature value selector 11 selects m feature values in which the distance between the first probability distribution and the second probability distribution is relatively small.

In step S15A, the feature value selector 11 generates and evaluates a tentative prediction model on the basis of the selected one or more feature values. That is, the feature value selector 11 executes the evaluation process. The "selected one or more feature values" are substantially the same as the "remaining one or more feature values" in step S10. Therefore, in step S15A, cross-validation shown in Figure 8 can be executed.

In step S16A, the feature value selector 11 determined whether the number of selections m is changed. The change of the number of selections m may be an increment or a decrement.

In an example, the feature value selector 11 determines whether the number of selections m is incremented, on the basis of the number of the selected feature values, or the number of feature values which have not been selected. In another example, the feature value selector 11 determines whether the number of selections m is decremented, on the basis of the number of the selected feature values, or the number of feature values which have not been selected.

When the number of selections m is changed (YES in step S16A), the process proceeds to step S17A. In step S17A, the feature value selector 11 changes the number of selections m by a predetermined number. The change is an increment or a decrement. The predetermined number is any positive integer. For example, the feature value selector 11 increases the number of selections m by 1, 2, 3, 4, 5, 6, 7, 8, 9 or 10. As another example, the feature value selector 11 increases the number of feature values selected by a number equal to the initial value of m. Alternatively, the feature value selector 11 reduces the number of selections m by 1, 2, 3, 4, 5, 6, 7, 8, 9 or 10. After step S17A, the process returns to step S14A. In step S14A that is repeated, the feature value selector 11 selects m feature values from a plurality of feature values on the basis of the selection index for each feature value. In step S15A that is repeated, the feature value selector 11 generates and evaluates a tentative prediction model on the basis of the selected one or more feature values. The feature value selector 11 gradually increases or reduces the number of selections m to calculate an evaluation index for the tentative prediction model for each number of selections m.

When the number of selections m is not changed (NO in step S16A), the process proceeds to step S18. In step S18, the feature value selector 11 selects one or more input feature values from a plurality of feature values. In an example, the feature value selector 11 determines the final number of selections m_{FINAL} on the basis of a final evaluation index obtained for each number of selections. The feature value selector 11 selects m_{FINAL} feature values from a plurality of feature values on the basis of the selection index for each feature value. When a distribution distance such as a Wasserstein distance is used, the feature value selector 11 selects, as input feature values, m_{FINAL} feature values in ascending order of the distribution distance. That is, the feature value selector 11 selects, as one or more input feature values, one or more feature values in which the distribution distance is smaller than a predetermined reference determined on the basis of the final evaluation index. As described above, the predetermined reference can be determined by a person or automatically.

As described above, the feature value selector 11 repeats an evaluation process in which generation of a tentative prediction model and calculation of an evaluation index for the tentative prediction model are executed on the basis of the selected one or more feature values while the number of feature values selected from a plurality of feature values on the basis of a selection index for each of a plurality of feature values is changed (steps S14A to S17A are repeated). The feature value selector 11 can execute the evaluation process by cross-validation. The feature value selector 11 selects one or more input feature values on the basis of a plurality of evaluation indices obtained by repeating the evaluation process.

### (Learning phase)

A process for generating the prediction model 30 on the basis of the selected one or more input feature values, that is, step S20 will be described. The learner 12 generates the prediction model 30 by executing machine learning (supervised learning) using one or more input feature values of training data acquired by the feature value selector 11. In the machine learning, the feature value selector 11 does not use the excluded n_{FINAL} feature values, and inputs one or more feature values of training data as input parameters (for example, input vectors) to the machine learning model. In an example, the feature value selector 11 updates a group of parameters in the machine learning model by executing backpropagation (a backpropagation method) based on an error between a predicted value calculated by the machine learning model and a correct solution (label). The learner 12 obtains the prediction model 30 by repeating the process until a given end condition is met. The end condition may be processing of all data records of training data (group of first molecules). It is to be noted that the prediction model 30 generated is a calculation model presumed to be optimal, and is not necessarily a "calculation model that is actually optimal".

### (Prediction phase)

Prediction using the prediction model 30, that is, step S30 will be described. The predictor 13 acquires one or more input feature values for each of one or more molecules of interest. Each molecule of interest can be a molecule specified through input operation or selection operation by a user of the information processing system 10. The predictor 13 may read an input feature value from a predetermined database or receive an input feature value from another computer such as a user terminal for each molecule of interest. The predictor 13 inputs one or more input feature values of the molecule of interest to the prediction model 30. When one or more other feature values are acquired in addition to one or more input feature values for the molecule of interest, the predictor 13 does not use the one or more other feature values, and inputs one or more input feature values as input parameters (for example, input vectors) to the prediction model 30. The prediction model 30 calculates feature values of the molecule of interest on the basis of the input feature values, and the predictor 13 acquires the feature values from the prediction model 30. The predictor 13 thus uses the prediction model 30 to acquire feature values for each of one or more molecules of interest.

The predictor 13 may output the feature values of each of one or more molecules of interest as prediction results. When a characteristic value is acquired for each of a plurality of molecules of interest, the predictor 13 may select at least one of a plurality of molecules of interest on the basis of the characteristic values. For example, the predictor 13 selects molecules of interest whose characteristic values meet a predetermined reference. Such selection can also be said to be screening. The predictor 13 may output, as a prediction result, information on at least one molecule of interest which is selected. The information may include at least one of a name, a structure, sequence information and a characteristic value of the molecule of interest.

In step S30, the predictor 13 can process at least one of a plurality of second molecules as a molecule of interest. The predictor 13 may acquire one or more feature values for each molecule of interest, and output, for each molecule of interest, a characteristic value of the molecule of interest which is obtained by inputting the acquired one or more input feature values to the generated prediction model. When a characteristic value is acquired for each of a plurality of second molecules, the predictor 13 may select at least one of a plurality of second molecules as a candidate molecule on the basis of the characteristic values, and output information on at least one candidate molecule which is selected.

The predictor 13 may store the prediction result in a storage device such as an auxiliary storage 103, may display a prediction result on an output device 106, or may send the prediction result to another computer such as a user terminal.

### (Method for producing molecular compound)

On the basis of information on at least one molecule of interest or candidate molecule output in the prediction phase, a molecular compound having a molecular sequence of the at least one molecule of interest or candidate molecule may be generated.

When the molecular compound is an antibody, the antibody can be produced by, for example, a method or a configuration of recombination as described in U.S. Patent No. 4,816,567. An example of the production method is a method for preparing an antibody, comprising culturing, under conditions suitable for expression of an antibody which is a candidate molecular compound described herein, host cells containing a nucleic acid encoding the antibody, or collecting the antibody from the host cells (or a host cell culture medium). The isolated nucleic acid encoding the antibody may encode an amino acid sequence containing VL of the antibody and/or an amino acid sequence containing VH of the antibody (for example, a light chain and/or a heavy chain of the antibody). Host cells containing such a nucleic acid contain (1) a vector containing a nucleic acid encoding an amino acid sequence containing VL of the antibody and an amino acid sequence containing VH of the antibody, or (2) a first vector containing a nucleic acid encoding an amino acid sequence containing VL of the antibody and a second vector containing a nucleic acid encoding an amino acid sequence containing VH of the antibody (for example, the host cells are transformed). In an example, host cells are eukaryotic (for example, Chinese hamster ovary (CHO) cells or lymphatic cells (for example, Y0, NS0, Sp2/0 cells)). Host cells suitable for cloning or expression of a vector encoding an antibody include prokaryotic cells or eukaryotic cells. For example, the antibody may be produced using bacteria particularly when glycosylation and an Fc effector function are not required. Antibody fragments and polypeptides can be expressed in bacteria by referring to, for example, US 5648237, US 5789199 and US 5840523. In addition, antibody fragments can be expressed in Escherichia coli by referring to "Charlton, Methods in Molecular Biology, Vol. 248 (B.K.C. Lo, ed., Humana Press, Totowa, NJ, 2003), pp. 245-254". After expression, the antibody may be isolated into soluble fractions from a bacterial cell paste, or further purified.

When the molecular compound is a peptide compound or a cyclic peptide compound, the compound can be produced by a liquid-phase synthesis method, a solid-phase synthesis method involving Fmoc synthesis, Boc synthesis or the like, or a combination thereof. The solid synthesis is a method in which a compound is bound to a solid, and the compound and a reagent is chemically reacted on the solid resin to synthesize an intended compound. The solid-phase synthesis of a peptide is a method in which a desired amino acid or peptide is bound to a solid resin, and to the amino acid or the peptide bound to the solid resin, desired amino acids or peptides are sequentially linked to extend the peptide chain, thereby synthesizing a peptide. By separating, from the solid resin, the peptide bound to the solid resin, an intended peptide is obtained.

### [Molecule]

The molecule may be a small molecule (low-molecular compound), a middle molecule (middle-molecular compound), or a macromolecule (macromolecular compound). In the present disclosure, the small molecule or low-molecular compound is a compound having a molecular weight of less than 500 g/mol. In the present disclosure, the middle molecule or middle-molecular compound is a compound having a molecular weight of 500 g/mol or more and less than 30,000 g/mol. In the present disclosure, the macromolecule or macromolecular compound is a compound having a molecular weight of 30,000 g/mol or more.

The molecule may be a biomolecule or a non-biomolecule. The molecule may be a nucleic acid, a peptide, a cyclic peptide, a protein, or an antigen-binding molecule such as an antibody, or a molecule that binds to a target molecule (target molecule-binding molecule). The molecule may be a drug candidate molecule. When the molecule is a peptide, a cyclic peptide, a protein or an antibody, the constituent units of the molecule are amino acids. When the molecule is a nucleic acid, the constituent units of molecules are nucleosides or nucleotides.

In the present disclosure, the desired characteristic is a characteristic required for a new target suitable for a drug candidate, and may be arbitrarily set. Examples of the characteristic include, but are not limited to, ability to bind to a predetermined *in vivo* target, pharmacological activity, physical properties, kinetics, and safety. Examples of physical properties include thermal stability, chemical stability, solubility, viscosity, photostability, long-term storage stability, non-specific adsorptivity, lipophilicity, and membrane permeability. As an example, when the molecule designed is mRNA (messenger-RNA), the characteristic is ability to be translated to a protein. The molecule is a molecule that binds to a target molecule, such as an antigen-binding molecule, and the characteristic may be ability to bind to the target molecule.

### - Antigen-Binding Molecule

In the present disclosure, the antigen-binding molecule containing an antigen-binding domain is used in the broadest sense. Specifically, the antigen-binding molecule includes various molecular types as long as it contains an antigen-binding domain. The antigen-binding molecule may be a molecule consisting only of an antigen-binding domain, or a molecule containing an antigen-binding domain and another domain. If the antigen-binding molecule is a molecule formed of an antigen-binding domain and an Fc region bound together, for example, examples of such molecules include complete antibodies and antibody fragments. Antibodies can include single monoclonal antibodies (including agonist and antagonist antibodies), human antibodies, humanized antibodies, and chimeric antibodies. The antigen-binding molecule in the present disclosure can include scaffold molecules formed as a library for construction of antigen-binding domains by using only a partial structure of a three-dimensional structure, as a scaffold, such as an existing stable α/β barrel protein structure.

### - Evaluation of Binding Ability

The technique of evaluation of ability of a target molecule-binding molecule to bind to a target molecule is not limited. The evaluation of binding ability can be made by quantitatively evaluating the binding of the target molecule-binding molecule to the target molecule. The target molecule is, for example, a target protein. The target molecule-binding molecule is, for example, an antigen-binding molecule, and the target molecule is, for example, an antigen. For example, when the target molecule is an antigen, evaluation can be made by measuring binding activity of an antigen-binding molecule and an antigen. The binding activity is the total intensity of non-covalent interactions between one or more binding sites of a molecule (e.g., an antibody) and a binding partner for the molecule (e.g., an antigen). Here, "binding activity" is not strictly limited to 1:1 interaction between members of a certain binding pair (e.g., an antibody and an antigen). When members of a binding pair reflect monovalent 1:1 interaction, for example, the meaning of binding activity is intrinsic binding affinity (sometimes referred to simply as "affinity"). If members of a binding pair are capable of both monovalent binding and multivalent binding, the binding activity is the sum of binding forces of them. The binding activity of a molecule, X, to its partner, Y, is generally represented by a dissociation constant (KD) or the "amount of analyte bound per unit amount of ligand". For example, the octet value is one of indices of binding ability, and is measured as an amount of analyte bound per unit amount of ligand. Binding activity can be measured with any of common methods known in the art including those described herein. Those skilled in the art can appropriately determine conditions except the concentration of a compound specific to target tissue.

In a mode, for the binding activity of an antibody, a ligand capture method, for example, with a BIACORE (R) T200 or BIACORE (R) 4000 (GE Healthcare, Uppsala, Sweden), which uses surface plasmon resonance analysis as the measurement principle, is used.

In a mode, a measurement result is analyzed using BIACORE (R) evaluation software. Calculation of kinetics parameters is carried out by simultaneously fitting sensorgrams for association and dissociation with use of a 1:1 binding model. Through the process, association rates (kon or ka), dissociation rates (koff or kd), and equilibrium dissociation constants (KD) can be calculated.

As the value of antigen-binding activity, K D (dissociation rate constant) can be used when the antigen is a soluble molecule, and an apparent k d (apparent dissociation rate constant) can be used when the antigen is a membrane-associated molecule. k d (dissociation rate constant) and apparent K D (apparent dissociation rate constant) can be measured by methods known to those skilled in the art. For example, Biacore (GE healthcare), a flow cytometer, or the like can be used.

Another mode of characterization is, for example, a selection technique for antigen-binding molecules with use of a display library. In a mode, an example is panning using phage display. In evaluation of affinity, for example, a phage presenting an antigen-binding molecule that interacts with a target antigen can be concentrated through a process in which a phage library presenting a plurality different antigen-binding molecules is prepared, a target antigen is brought into contact with the phages prepared, and an operation to wash out unbound phages is then carried out. A sequence having affinity with the target antigen can be identified by analyzing the nucleic acid sequence encoding the antigen-binding molecule contained in the concentrated phage. In a mode, an example is panning using mammalian cell display. In evaluation of pharmacological activity using the display system, cells having an antigen-binding molecule gene with desired pharmacological activity can be isolated by using a flow cytometer or the like through a process in which a library containing a plurality of different antigen-binding molecules is expressed in targeted mammalian cells and reporter activity and so on are changed according to the action exhibited thereby on the cells. In evaluation of physical properties using the display system, cells having an antigen-binding molecule gene capable of stably expressing at high level can be isolated by using a flow cytometer or the like through a process in which a library containing a plurality of different antigen-binding molecules is expressed in targeted mammalian cells and the expression levels are examined by staining with antibodies specific to the antigen-binding molecules. Characterization of antigen-binding molecules by panning is not limited to the techniques using phages or mammalian cells, and various techniques can be used as long as they allow presentation of antigen-binding molecules. Examples thereof include a technique to allow ribosomes to present, a technique to allow mRNA to present, a technique to allow viruses other than phages to present, and a technique to allow bacteria such as Escherichia coli to present.

Another mode of characterization is, for example, a method of obtaining an antibody gene sequence from immunocytes derived from an individual or a method of obtaining an antibody protein sequence from serum. In evaluation of affinity involving extraction of an antibody gene sequence from immunocytes, a sequence having affinity with a target antigen can be identified through a process involving administering a target antigen protein to an individual to induce immune sensitization, and extracting an antibody gene for an antibody that binds to the target antigen from immunocytes having the gene.

For the antigen to cause immune sensitization, not only the technique using a protein, but also a technique using a gene encoding the protein, or cells expressing the protein may be used.

Examples of the individual as a subject include humans, mice, rats, hamsters, rabbits, monkeys, chickens, camels, llamas, and alpacas, but are not limited thereto.

Examples of techniques for the analysis of nucleic acid sequences or appearance frequency include a technique in which gene recombinant organisms having nucleic acid sequences for different antigen-binding molecules are cloned and analysis is performed through the Sanger method using capillary electrophoresis, and a technique to analyze with use of a next-generation sequencer, but are not limited thereto.

In the analysis of nucleic acid sequences, strength of a characteristic may be determined on the basis of appearance frequency. For example, the characteristic of an antigen-binding molecule encoded by a sequence with high appearance frequency in analysis of nucleic acid sequences after concentration can be estimated to be high. On the other hand, the characteristic of an antigen-binding molecule encoded by a sequence with low appearance frequency after concentration can be estimated to be lower than that of an antigen-binding molecule encoded by a sequence with high appearance frequency.

The techniques to acquire information on antigen-binding molecules derived from the display library or an individual are applicable to various types of characterization other than those described above.

### - Evaluation of Pharmacological Activity

The technique of evaluation of the pharmacological activity of molecules is not limited. The pharmacological activity can be evaluated by, for example, measuring neutralization activity, agonist activity, or cytotoxic activity exhibited by molecules. Examples of the evaluation of cytotoxic activity which is a type of evaluation of pharmacological activity include evaluations of antibody-dependent cell-mediated cytotoxic (ADCC) activity, complement-dependent cytotoxic (CDC) activity, T-cell-dependent cytotoxic (TDCC) activity, and antibody-dependent cellular-phagocytotic (ADCP) activity. CDC activity is cytotoxic activity due to the complement system. ADCC activity is such activity that an immunocyte binds to the Fc region of an antigen-binding molecule containing an antigen-binding domain that binds to a membrane-associated molecule expressed on the cell membrane of a target cell via the Fcy receptor expressed on the immunocyte, and the immunocyte causes damage to the target cell. TDCC activity is such activity that a T cell causes damage to a target cell through bringing the target cell and the T cell into close proximity with use of a bi-specific antibody containing an antigen-binding domain that binds to a membrane-associated molecule expressed on the cell membrane of the target cell and an antigen-binding domain against any of the constituent subunits of the T cell receptor (TCR) complex on the T cell (in particular, an antigen-binding domain that binds to the CD3 epsilon chain). Whether an antigen-binding molecule of interest has ADCC activity, CDC activity, TDCC activity, or ADCP activity can be determined by a known method.

Neutralization activity is activity that is against ligands having biological activity against cells (for example, viruses and toxins), and inhibits the biological activity. That is, the substance having neutralization activity is a substance that binds to a ligand or a receptor to which the ligand binds to inhibit binding between the ligand and the receptor. A receptor that has been inhibited from binding to the ligand by neutralization activity becomes unable to exhibit the biological activity mediated by the receptor. Neutralization activity is not limited to cases with inhibition of binding between a ligand and a receptor, and activity to inhibit the function of a protein having biological activity is also understood as neutralization activity. Examples of the function of the protein include enzymatic activity.

### - Evaluation of Physical Properties

As an example, evaluation of stability such as thermal stability, chemical stability, photostability, stability to mechanical stimulation, and long-term storage stability can be made by measuring the decomposition, chemical modification and association of a molecule before and after treatment for which the evaluation of stability is intended, such as heat treatment, exposure to a low-pH environment, exposure to light, stirring with a machine, and long-term storage. Examples of the measurement method for the evaluation of stability include techniques involving chromatography such as ion-exchange chromatography or size exclusion chromatography, mass spectrometry, and electrophoresis. Another measurement method may be used.

Other examples of evaluation of physical properties include evaluation of solubility of protein by a polyethylene glycol precipitation method, evaluation of viscosity by a small-angle X-ray scattering method, and evaluation of non-specific binding based on evaluation of binding to an extracellular matrix (ECM).

Still other examples of evaluation of physical properties include evaluation of a protein expression level, evaluation of binding to a resin for purification or a ligand for purification, and evaluation of surface electric charge.

### - Evaluation of Kinetics

Evaluation of kinetics of a molecule can be made by administering the molecule to an animal such as a mouse, a rat, a monkey or a dog and measuring the amount of the molecule in the blood after administration over time. Alternatively, evaluation of kinetics can be made by pharmacokinetics (PK) evaluation. As a technique other than direct evaluation of PK, the behavior of a molecule in kinetics may be predicted from the amino acid sequence of a molecule by calculating the surface electric charge, isoelectric point, and so on of the molecule with software.

### - Evaluation of Safety

Examples of evaluation of safety of a molecule include an immunogenicity prediction tool such as ISPRI Web-Based Immunogenicity Screening (EpiVax, Inc.), evaluation of HLA binding of fragment peptides of an antigen-binding molecule, MAPs (MHC-associated peptide proteomics), and detection of a T-cell epitope and evaluation of immunogenicity which involve evaluation of T-cell growth or the like. Evaluation of safety can be made as long as measurement can be performed by a technique such as evaluation of immunoreaction using binding to a rheumatoid factor (RF), PBMC, or whole blood and evaluation of platelet aggregation.

In an example, a drug discovery system comprising the information processing system 10 provides a method for generating a new target having a predetermined characteristic such as specific physiological activity (for example, binding to a specific protein). Examples of the drug include potential activators such as low-molecular pharmaceuticals, medium-molecular pharmaceuticals, biological agents, cells, nucleic acid pharmaceuticals, biopharmaceuticals, and other activators. The target includes a molecular structure having desired or defined biological activity. The biological activity may be, for example, binding to a specific protein in preference to other proteins. Molecules as drug candidates include biological molecules, and compounds, with the inclusion of various molecules such as nucleic acids, peptides, cyclic peptides, proteins, antibodies, target molecule-binding molecules, high-molecular compounds, medium-molecular compounds, and low-molecular compounds.

The drug discovery system may comprise a device for selecting a molecule that interacts with a target for an agent, and a device for creating a lead molecule, and the like. The drug delivery system may be, for example, an information processing system comprising the disclosure of WO 2020/246617.

The drug discovery system comprises a molecular design device. The molecular design device searches for a candidate of a molecule having a desired characteristic, and outputs information on the identified candidate molecule. The drug discovery system selects a new target suitable for a drug candidate using information on the candidate molecule which has been output from the molecular design device.

### [Validation Example]

### (First Validation Example)

Next, validation examples for an information processing system according to the present disclosure will be described.

In a first validation example, the usefulness of the information processing system according to the present disclosure was validated using a data set representing a measured value of a binding force of each of a plurality of antibody sequences. The data set comprises the sequence of a bispecific antibody which recognizes MarvelD3 and CD3 as antigens, and the characteristic value of binding ability. The theme of the first validation example was to improve the binding ability of the antibody with MarvelD3 assumed as an antigen. An octet value was used as the characteristic value of binding ability.

MarvelD3 is a tight junction protein having a 4-transmembrane structure. In the first validation example, MarvelD3 was set as a target candidate for an anticancer drug. The development of a bispecific antibody that bridges a cancer antigen and an antigen on T cells is expected to be applied to cancer treatment. In the first validation example, the learned prediction model was used when a candidate of an anti-MarvelD3 sequence having a better characteristic was determined from lead antibodies.

In the first validation example, a plurality of measurements of the octet value of an antibody sequence were made, and the antibody sequence and the octet value of the antibody sequence, which had been obtained by the measurement, were used as inputs to the prediction model. By batch measurement, the octet values typically of 100 or fewer antibody sequences are acquired in one measurement.

An antibody used in the measurement was acquired by the following procedure. First, a plasmid encoding a heavy chain or a light chain designed in advance was provided, and a recombinant antibody was transiently expressed using Expi293F cells. The antibody was taken from a culture supernatant using protein A, and the antibody was dissolved in a buffer solution. The dissolving buffer solutions were mixed under reducing conditions to prepare a MarvelD3/CD3 bispecific antibody. In the preparation, charge repulsion was applied between identical heavy chains to perform selective heavy chain heterodimerization. The concentration of the antibody in the buffer solution was determined by an absorbance at 280 nm. Thereafter, ion-exchange chromatography was performed on the buffer solution containing a bispecific antibody, and it was confirmed that an intended MarvelD3/CD3 antibody was provided.

An Octet HTX system was used for measurement of the octet value. Extracellular vesicles having a CD81 protein and a human MarvelD3 on surfaces thereof were caught on a sensor chip using an anti-CD81 antibody. After a 600-second baseline step in a D-PBS(-) solution containing 0.1% BSA, an association response and a dissociation response in identical buffer solutions containing 20 nM of the antibody were measured for 900 seconds and 1,500 seconds, respectively. The binding ability of the antibody appears as a wavelength change between the baseline step and the end of the association stage. The measurement was performed with a sample plate vibrated at a temperature of 30°C and a vibration rate of 1,000 times per minute in the stages of the baseline step, association and dissociation.

A specific method is shown below. First, 1,144 antibody sequences were extracted as a group of sequences for training. Next, a characteristic prediction model for predicting a binding force was prepared using a training data representing the group of sequences for training. A group of virtual sequences comprising 196,608 virtual sequences prepared by combination of candidates for amino acid alteration and having no measure values was input as a group of sequences for prediction to the characteristic prediction model. The group of sequences for prediction was screened on the basis of a prediction value of the characteristic prediction model for each antibody sequence contained in the group of sequences for prediction, and whether it was possible to select an antibody sequence with a binding force was validated.

In preparation of the characteristic prediction model, each antibody sequence was converted into a vector of 768 dimensions for each of a VH region and a VL region using a protein language model TAPE, and vector data of 1,536 dimensions which had been obtained by combination of the vectors was defined as feature values of each antibody sequence.

For validating the usefulness of the present invention, the input feature value for use in generation of the prediction model was selected from the feature values of 1536 dimensions by the following two types of methods. When a characteristic prediction model was constructed using the selected feature value, the levels of the binding forces of antibody sequences selected with the model were compared. In the first method, LightGBM (light gradient-boosting machine) is used. In the first method, a class classification model for discriminating a group of sequences for training and a group of sequences for prediction is prepared, and on the basis of the feature importance obtained from the model, the importance of each dimension in the feature values of 1,536 dimension is calculated. In the first method, feature values are selected so as to increase the number 10 by 10 in order from a feature value which is of low importance, that is, a feature value which is estimated to be small in difference between the group of sequences for training and the group of sequences for prediction. In the second method, the distribution distance is defined by a Wasserstein distance for each of the dimensions of feature values of each of the group of sequences for training and the group of sequences for prediction. In the second method, feature values are selected so as to increase the number in order from a feature value which is small in Wasserstein distance, that is, a feature value which is relatively consistent in distribution between the group of sequences for training and the group of sequences for prediction. A cross-validation method using a mean square error as an index was applied to each set of feature values selected stepwise by each of the first and second methods, thereby identifying a set of feature values at which prediction performance against the group of sequences for training was the highest. Using the set of feature values, performance of characteristic prediction of the binding force by LightGBM was evaluated.

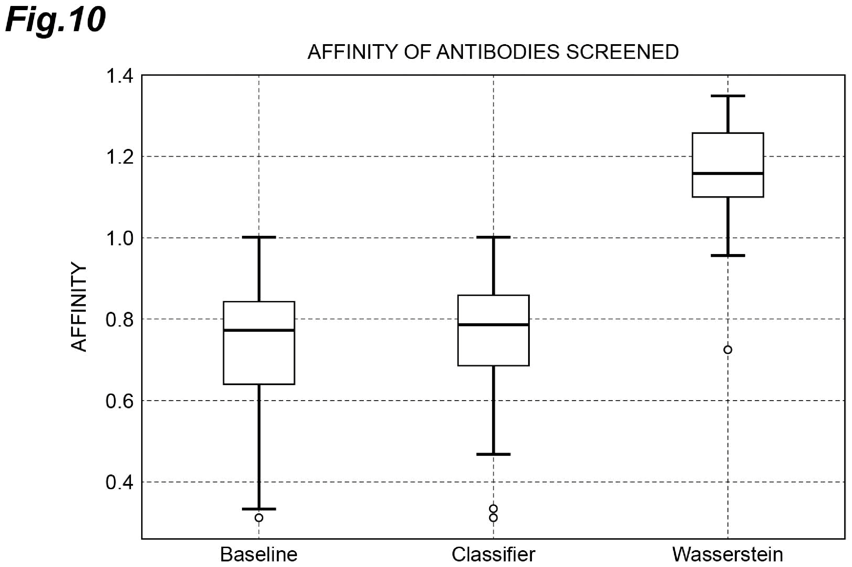

Figure 10 shows a distribution of measured values of antibody sequences selected as top 16 antibody sequences when selected in descending order of the predicted value of the binding force from the group of sequences for prediction for each of cases where feature value selection was not performed and all feature values were used as input feature values (Baseline), where feature values selected using feature importance obtained from the class classification model (Classifier), and where feature values selected using a Wasserstein distance (Wasserstein). Figure 10 demonstrates that the use of feature values selected using a Wasserstein distance enabled screening antibody sequences with a higher measured value of the binding force as compared to the use of all feature values and the use of feature values selected using feature importance. That is, Figure 10 demonstrates that the information processing system (feature value selection technique) according to the present disclosure is useful.

### (Second Validation Example)

Next, a second validation example will be described. In the second validation example, the usefulness of the information processing system according to the present disclosure using AqSoIDB which is a data set of the solubility of 9,982 low-molecular compounds and which was downloaded using Therapeutics Data Common that is a platform for providing the function of downloading public data sets. Using 1,000 molecules randomly extracted from all compounds as a group of first molecules, a characteristic prediction model for predicting solubility was prepared. Next, using remaining 8,982 molecules as a group of second molecules, a group of second molecules was screened on the basis of the predicted value of the characteristic prediction model. This was followed by validation of whether it was possible to select molecules with a high measured value of solubility. For the validation, the number of types of random number sequences when molecules were randomly extracted was set to 10, and the validation was performed 10 times. In the validation, feature values were selected in accordance with the step S10A. In step S11, training data and test data including feature values of 2,048 dimensions extracted using MolCLR (Molecular Contrastive Learning of Representations via Graph Neural Networks) were acquired. In steps S12 and S14A, the Wasserstein distance and the feature importance of the model for making classification between the group of first molecules and the group of second molecules were each used, and the two types of selection indices were compared. In step S13A, the initial value of the number of selections m of feature values was set to 18. In step S15A, the cross-validation method was used as a method for evaluating the performance of the tentative prediction model, the mean square error was used as an index for evaluation of the performance, and LightGBM (light gradient-boosting machine) was used as a technique of supervised learning. In step S16A, it was determined that the process proceeded to step S17A when the number of selections m of feature values was less than 2,048, and the process proceeded to step S18 when the number of selections m was 2,048. In step S17A, the number of selections m of feature values was incremented by 10. In step S18, a set of feature values where the average value of mean square errors of the tentative prediction model was the smallest was selected as input feature values.

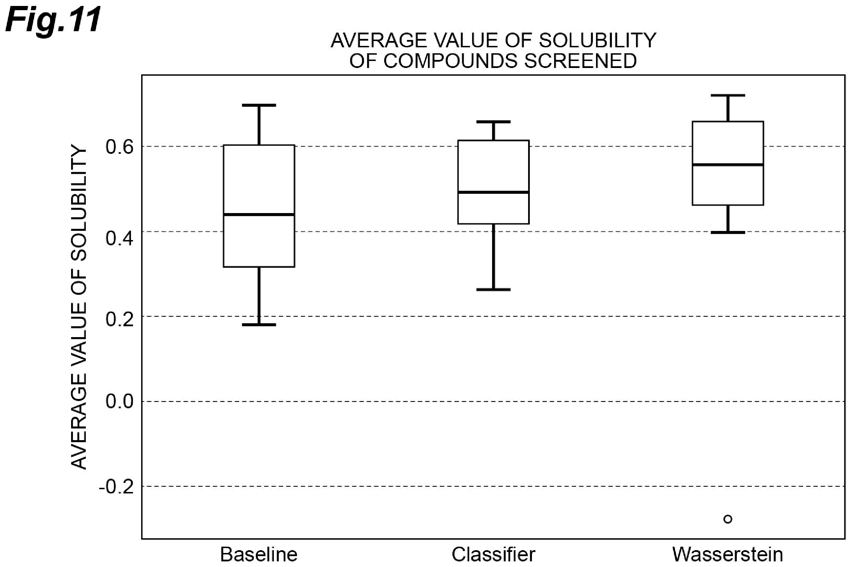

Figure 11 shows a distribution of averages of measured values of the solubility of compounds selected as top 100 compounds when selected in descending order of the predicted value of the solubility for each of cases where feature value selection was not performed and all feature values were used as input feature values (Baseline), where feature values selected using feature importance as a selection index for feature values (Classifier), and where feature values selected using a Wasserstein distance as a selection index for feature values (Wasserstein). Figure 11 demonstrates that the use of feature values selected using a Wasserstein distance enabled screening compounds with a higher measured value of solubility as compared to the use of all feature values and the use of feature values selected using feature importance. That is, Figure 11 demonstrates that the information processing system (feature value selection technique) according to the present disclosure is useful.

### [Modifications]

The technique according to the present disclosure has been described in detail above on the basis of various examples thereof. However, the present disclosure is not limited to the above examples. Various modifications of the technique according to the present disclosure can be made without departing from the spirit of the present disclosure.

In the above examples, the information processing system 10 comprises the learner 12 and the predictor 13 in addition to the feature value selector 11, but the information processing system may be free of at least one of the learner and the predictor. The prediction model can be transplanted between computer systems. Therefore, a prediction model generated by the information processing system may be used in another computer system. Alternatively, the other computer system may generate a prediction model through machine learning using one or more input feature values selected by the information processing system, with the information processing system executing a prediction phase using the prediction model.

The information processing system may be constructed as a server in a client server system, or implemented in a stand-alone computer. Alternatively, the information processing system may be implemented in a user terminal capable of accessing, through a communication network, a database for storing training data and test data.

The procedure of a method executed by at least one processor is not limited to the above examples. For examples, a part of the steps or processes described above may be omitted, and the steps may be executed in another order. Any two or more of the steps described above may be combined, and a part of the steps may be modified or deleted. Alternatively, in addition to the steps described above, other steps may be executed.

In comparison of the magnitudes of two numerical values in the present disclosure, any of two criteria, i.e., "or more" and "more than" criteria may be used, and any of two criteria, i.e., "or less" and "less than" criteria may be used.

In the present disclosure, the wording "at least one processor executes a first process, executes a second process, ..., and executes an nth process", or equivalent wording indicates a concept including a case where the processor to execute n processes from the first to nth processes changes in the middle. That is, this wording indicates a concept including both a case where all n processes are executed by the same processor and a case where the processor is changed in n processes due to any policy.

In the present disclosure, the term "to" that indicates a range includes values of both ends thereof. For example, "A to B" means the range of A or more and B or less.

In the present disclosure, the term "about" when used in combination with a numerical value means a range between +10% and -10% of the numerical value.

In the present specification, the term "and/or" is used to show the matters described before and after "and/or", or any combination thereof. For example, "A, B and/or C" includes the matters "A", "B" and "C", and combinations "A and B", "A and C", "B and C", and "A and B and C".

### [Supplements]

As is understood from the various examples described above, the present disclosure includes the following aspects.

### (Supplement 1)

An information processing system comprising at least one processor,
wherein the at least one processor
acquires, for each of a plurality of first molecules, training data representing a plurality of feature values related to the first molecule,
acquires, for each of a plurality of second molecules, test data representing the plurality of feature values related to the second molecule,
calculates, for each of the plurality of feature values, an index based on a first probability distribution which is a probability distribution in the training data and a second probability distribution which is a probability distribution in the test data, and
selects, on the basis of the index of each of the plurality of feature values, one or more of the plurality of feature values as one or more input feature values to be used as input parameters of a prediction model for predicting a characteristic value of a molecule on the basis of machine learning.

### (Supplement 2)

The information processing system according to supplement 1,
wherein the at least one processor
calculates, for each of the plurality of feature values, a distribution distance, which is a distance between the first probability distribution and the second probability distribution, as the index, and
selects the one or more input feature values from the plurality of feature values on the basis of the distribution distance for each of the plurality of feature values.

### (Supplement 3)

The information processing system according to supplement 2, wherein the at least one processor selects, as the one or more input feature values, the one or more feature values in which the distribution distance is smaller than a predetermined reference.

### (Supplement 4)

The information processing system according to supplement 2 or 3, wherein the at least one processor calculates a Wasserstein distance as the distribution distance.

### (Supplement 5)

The information processing system according to any one of supplements 2 to 4,
wherein the at least one processor
excludes a part of the plurality of feature values on the basis of the distribution distance for each of the plurality of feature values, and selects the remaining one or more feature values as the one or more input feature values.

### (Supplement 6)

The information processing system according to supplement 5,
wherein the at least one processor
repeats an evaluation process in which generation of a tentative prediction model by the machine learning using the training data and calculation of an evaluation index for the tentative prediction model are executed on the basis of the remaining one or more feature values while changing the number of feature values excluded from the plurality of feature values on the basis of the distribution distance for each of the plurality of feature values,
determines, on the basis of a plurality of the evaluation indices, the number of feature values excluded from the plurality of feature values, as the number of exclusions, and
excludes the feature values by the number of exclusions from the plurality of feature values on the basis of the distribution distance for each of the plurality of feature values, and selects the one or more input feature values.

### (Supplement 7)

The information processing system according to supplement 6,
wherein the plurality of feature values are N feature values, where N is a positive integer of 2 or more, and
the at least one processor
   sets the number of feature values excluded from the N feature values to n, where n is a positive integer,
   excludes n feature values from the plurality of feature values on the basis of the distribution distance for each of the N feature values,
   performs an evaluation process in which generation of a first tentative prediction model by the machine learning using the training data and calculation of an evaluation index for the first tentative prediction model are executed on the basis of (N-n) feature values,
   further excludes n feature values from the (N-n) feature values on the basis of the distribution distance for each of the (N-n) feature values, and
   performs an evaluation process in which generation of a second tentative prediction model by the machine learning using the training data and calculation of an evaluation index for the second tentative prediction model are executed on the basis of (N-2n) feature values.

### (Supplement 8)

The information processing system according to supplement 6 or 7, wherein the at least one processor determines, as the number of exclusions, the number at which the highest evaluation index is obtained.

### (Supplement 9)

The information processing system according to supplement 6 or 7, wherein the at least one processor determines, as the number of exclusions, the number at which the lowest evaluation index is obtained.

### (Supplement 10)

The information processing system according to supplement 5,
wherein the at least one processor
repeats an evaluation process in which generation of a tentative prediction model by the machine learning using the training data and calculation of an evaluation index for the tentative prediction model are executed on the basis of the selected one or more feature values while changing the number of feature values selected from the plurality of feature values on the basis of the distribution distance for each of the plurality of feature values,
determines, on the basis of a plurality of the evaluation indices, the number of feature values selected from the plurality of feature values, as the number of selections, and
selects the feature values by the number of selections from the plurality of feature values as the one or more input feature values on the basis of the distribution distance for each of the plurality of feature values.

### (Supplement 11)

The information processing system according to supplement 10,
wherein the plurality of feature values are N feature values, where N is a positive integer of 2 or more,
the at least one processor
sets the number of feature values selected from the N feature values to m, where m is a positive integer,
selects m feature values from the plurality of feature values on the basis of the distribution distance for each of the N feature values,
performs an evaluation process in which generation of a first tentative prediction model by the machine learning using the training data and calculation of an evaluation index for the first tentative prediction model are executed on the basis of m feature values,
further adds m feature values to the m feature values on the basis of the distribution distance for each of the m feature values, and
performs an evaluation process in which generation of a second tentative prediction model by the machine learning using the training data and calculation of an evaluation index for the second tentative prediction model are executed on the basis of 2m feature values.

### (Supplement 12)

The information processing system according to supplement 11, wherein the at least one processor determines, as the number of selections, the number at which the highest evaluation index is obtained.

### (Supplement 13)

The information processing system according to supplement 11, wherein the at least one processor determines, as the number of selections, the number at which the lowest evaluation index is obtained.

### (Supplement 14)

The information processing system according to any one of supplements 6 to 13, wherein the at least one processor executes the evaluation process through cross-validation.

### (Supplement 15)

The information processing system according to any one of supplements 1 to 14, wherein the at least one processor generates the prediction model by executing the machine learning using the one or more input feature values of the training data.

### (Supplement 16)

The information processing system according to supplement 15,
wherein the at least one processor
acquires the one or more input feature values for a molecule of interest whose characteristic value is unknown, and
outputs the characteristic value of the molecule of interest which is obtained by inputting the acquired one or more input feature values to the generated prediction model.

### (Supplement 17)

The information processing system according to supplement 15,
wherein the at least one processor
acquires the one or more input feature values for each of a plurality of molecules of interest whose characteristic value is unknown,
inputs the acquired one or more input feature values to the generated prediction model for each of the plurality of molecules of interest, and acquires the characteristic value of the molecule of interest from the prediction model,
selects at least one molecule of interest among the plurality of molecules of interest on the basis of the characteristic value of each of the plurality of molecules of interest, and
outputs information on the at least one selected molecule of interest.

### (Supplement 18)

The information processing system according to any one of supplements 1 to 17, wherein each of the first molecules and the second molecules is one selected from a nucleic acid, a peptide, a cyclic peptide, a protein, an antibody, and a low-molecular compound.

### (Supplement 19)

The information processing system according to supplement 15,
wherein the at least one processor
acquires the one or more input feature values for each of at least one second molecule among the plurality of second molecules, and
outputs, for each of the at least one second molecule, the characteristic value of the second molecule which is obtained by inputting the acquired one or more input feature values to the generated prediction model.

### (Supplement 20)

The information processing system according to supplement 15,
wherein the at least one processor
acquires the one or more input feature values for each of the plurality of second molecules,
inputs the acquired one or more input feature values to the generated prediction model for each of the plurality of second molecules, and acquires the characteristic value of the molecule of interest from the prediction model,
selects, as a candidate molecule, at least one second molecule among the plurality of second molecules on the basis of the characteristic value of each of the plurality of second molecules, and
outputs information on the at least one selected candidate molecule.

### (Supplement 21)

The information processing system according to any one of supplements 1 to 20, wherein the characteristic value is selected from at least one of affinity, pharmacological activity, a physical property, kinetics, and safety.

### (Supplement 22)

The information processing system according to any one of supplements 1 to 20,
wherein the first molecule and the second molecule are antigen-binding molecules, and
the characteristic value is a value of ability of the antigen-binding molecule to bind to an antigen.

### (Supplement 23)

An information processing method executed by an information processing system comprising at least one processor, the method comprising the steps of:
acquiring, for each of a plurality of first molecules, training data representing a plurality of feature values related to the first molecule;
acquiring, for each of a plurality of second molecules, test data representing the plurality of feature values related to the second molecule;
calculating, for each of the plurality of feature values, an index based on a first probability distribution which is a probability distribution in the training data and a second probability distribution which is a probability distribution in the test data; and
selecting, on the basis of the index of each of the plurality of feature values, one or more of the plurality of feature values as one or more input feature values to be used as input parameters of a prediction model for predicting a characteristic value of a molecule on the basis of machine learning.

### (Supplement 24)

An information processing program that allows a computer to execute the steps of:
acquiring, for each of a plurality of first molecules, training data representing a plurality of feature values related to the first molecule;
acquiring, for each of a plurality of second molecules, test data representing the plurality of feature values related to the second molecule;
calculating, for each of the plurality of feature values, an index based on a first probability distribution which is a probability distribution in the training data and a second probability distribution which is a probability distribution in the test data; and
selecting, on the basis of the index of each of the plurality of feature values, one or more of the plurality of feature values as one or more input feature values to be used as input parameters of a prediction model for predicting a characteristic value of a molecule on the basis of machine learning.

### (Supplement 25)

A method for producing a molecular compound, comprising a generation step of generating a molecular compound having a molecular sequence of the at least one molecule of interest, on the basis of the information of the at least one molecule of interest which is output from the information processing system according to supplement 17.

### (Supplement 26)

A method for producing a molecular compound, comprising a generation step of generating a molecular compound having a molecular sequence of the at least one candidate molecule, on the basis of the information of the at least one candidate molecule which is output from the information processing system according to supplement 20.

### (Supplement 27)

A non-transitory and computer-readable recording medium configured to store an information processing program that allows a computer to execute the steps of:
acquiring, for each of a plurality of first molecules, training data representing a plurality of feature values related to the first molecule;
acquiring, for each of a plurality of second molecules, test data representing the plurality of feature values related to the second molecule;
calculating, for each of the plurality of feature values, an index based on a first probability distribution which is a probability distribution in the training data and a second probability distribution which is a probability distribution in the test data; and
selecting, on the basis of the index of each of the plurality of feature values, one or more of the plurality of feature values as one or more input feature values to be used as input parameters of a prediction model for predicting a characteristic value of a molecule on the basis of machine learning.

According to supplements 1, 23, 24 and 27, feature values to be used as input parameters of a prediction model based on machine learning are selected as input feature values on the basis of the tendency of each feature value in both training data and test data. By selecting feature values as described above, the accuracy of a machine learning model (prediction model) for predicting characteristics of a molecule can be improved. The improvement of the accuracy of the machine learning model can lead to prediction of the characteristics of a molecule with good accuracy.

According to supplement 2, feature values are selected on the basis of a distribution distance between the first probability distribution and the second probability distribution. By introducing a distribution distance representing a quantified difference in tendency between two probability distributions, input feature values can be selected on the basis of an objective criteria. As a result, further improvement of the accuracy of the machine learning model can be expected.

According to supplement 3, feature values which are relatively small in distribution distance are selected as input feature values. Input feature values selected as described above are relatively consistent in tendency between training data and test data. Therefore, the accuracy of the machine learning model (prediction model) can be improved by machine learning based on the input feature values.

According to supplement 4, the distribution distance can be appropriately calculated by introducing a Wasserstein distance as the distribution distance. As a result, input feature values can be more appropriately selected.

According to supplement 5, input feature values can be selected by focusing attention on feature values which are estimated to be unsuitable as input feature values.

According to supplement 6, generation and evaluation of the tentative prediction model are repeated while changing the number of feature values excluded, and the number of exclusions is dynamically determined on the basis on the basis of a plurality of evaluation indices obtained by the repetition. The feature values are excluded by the determined number of exclusions to select input feature values. In an example, if an excessive number of feature values which are relatively large in distribution distance are excluded, even feature values which can actually contribute to prediction of a characteristic value may be excluded. By dynamically determining the number of exclusions as described above, one or more input feature values for improving the accuracy of the machine learning model can be appropriately selected.

According to supplement 7, the ratio of increase in number of exclusions is constant in the repetition of the evaluation process, and therefore, the evaluation process can be efficiently repeated with a convenient technique.

According to supplement 8, the number of exclusions of feature values at which the highest evaluation index is obtained is determined as the final number of exclusions. According to supplement 9, the number of exclusions of feature values at which the lowest evaluation index is obtained is determined as the final number of exclusions. Therefore, it is possible to select one or more input feature values which are expected to make the machine learning model have the highest accuracy.

According to supplement 10, generation and evaluation of the tentative prediction model are repeated while the number of feature values selected is changed, and the number of selections is dynamically determined on the basis on the basis of a plurality of evaluation indices obtained by the repetition. Input feature values are selected by the determined number of selections. In an example, if an excessive number of feature values which are relatively small in distribution distance are selected, even feature values which do not actually contribute to prediction of a characteristic value may be selected. By dynamically determining the number of selections as described above, one or more input feature values for improving the accuracy of the machine learning model can be appropriately selected.

According to supplement 11, the ratio of increase in number of selections is constant in the repetition of the evaluation process, and therefore, the evaluation process can be efficiently repeated with a convenient technique.

According to supplement 12, the number of selections of feature values at which the highest evaluation index is obtained is determined as the final number of selections. According to supplement 13, the number of selections of feature values at which the lowest evaluation index is obtained is determined as the final number of selections. Therefore, it is possible to select one or more input feature values which are expected to make the machine learning model have the highest accuracy.

According to supplement 14, cross-validation is introduced in the evaluation processes for obtaining the evaluation indices, and therefore, even when the data amount of training data is limited, each tentative prediction model can be accurately evaluated by making effective use of the limited data.

According to supplement 15, machine learning for generating a prediction model is executed using one or more input feature values selected on the basis of the tendency of each of feature values in both training data and test data. Therefore, a prediction model with good accuracy can be obtained.

According to supplement 16, the characteristics of a molecule of interest can be predicted with good accuracy by using a prediction model generated by machine learning using the selected one or more input feature values.

According to supplement 17, the characteristics of each molecule of interest can be predicted with good accuracy by using a prediction model generated by machine learning using the selected one or more input feature values. Therefore, at least one molecule of interest can be appropriately selected from a plurality of molecules of interest.

According to supplement 18, the accuracy of a machine learning model (prediction model) for predicting the characteristics of a nucleic acid, a peptide, a cyclic peptide, a protein, an antibody and a low-molecular compound can be improved.

According to supplement 19, the characteristics of the second molecule can be predicted with good accuracy by using a prediction model generated by machine learning using the selected one or more input feature values.

According to supplement 20, the characteristics of each second molecule can be predicted with good accuracy by using a prediction model generated by machine learning using the selected one or more input feature values. Therefore, at least one candidate molecule can be appropriately selected from a plurality of candidate molecules.

According to supplement 21, the accuracy of a machine learning model (prediction model) for predicting affinity, pharmacological activity, physical properties, kinetics or safety of a molecule can be improved.

According to supplement 22, the accuracy of a machine learning model (prediction model) for predicting ability of an antigen-binding molecule to bind to an antigen.

According to supplement 25, a molecular compound which can be expected to have desired characteristics can be generated on the basis of information on at least one molecule of interest which is appropriately selected using a prediction model.

According to supplement 26, a molecular compound which can be expected to have desired characteristics can be generated on the basis of information on at least one candidate molecule which is appropriately selected using a prediction model.

### [Reference Signs List]

10...information processing system, 11...feature value selector, 12...learner, 13...predictor, 20...database, 30...prediction model, 110...information processing program, 210...first probability distribution, 220...second probability distribution

## Claims

1. An information processing system comprising at least one processor,
wherein the at least one processor
acquires, for each of a plurality of first molecules, training data representing a plurality of feature values related to the first molecule,
acquires, for each of a plurality of second molecules, test data representing the plurality of feature values related to the second molecule,
calculates, for each of the plurality of feature values, an index based on a first probability distribution which is a probability distribution in the training data and a second probability distribution which is a probability distribution in the test data, and
selects, on the basis of the index of each of the plurality of feature values, one or more of the plurality of feature values as one or more input feature values to be used as input parameters of a prediction model for predicting a characteristic value of a molecule on the basis of machine learning.

2. The information processing system according to claim 1,
wherein the at least one processor
calculates, for each of the plurality of feature values, a distribution distance, which is a distance between the first probability distribution and the second probability distribution, as the index, and
selects the one or more input feature values from the plurality of feature values on the basis of the distribution distance for each of the plurality of feature values.

3. The information processing system according to claim 2, wherein the at least one processor selects, as the one or more input feature values, the one or more feature values in which the distribution distance is smaller than a predetermined reference.

4. The information processing system according to claim 2 or 3, wherein the at least one processor calculates a Wasserstein distance as the distribution distance.

5. The information processing system according to any one of claims 2 to 4,
wherein the at least one processor
excludes a part of the plurality of feature values on the basis of the distribution distance for each of the plurality of feature values, and selects the remaining one or more feature values as the one or more input feature values.

6. The information processing system according to claim 5,
wherein the at least one processor
repeats an evaluation process in which generation of a tentative prediction model by the machine learning using the training data and calculation of an evaluation index for the tentative prediction model are executed on the basis of the remaining one or more feature values while changing the number of feature values excluded from the plurality of feature values on the basis of the distribution distance for each of the plurality of feature values,
determines, on the basis of a plurality of the evaluation indices, the number of feature values excluded from the plurality of feature values, as the number of exclusions, and
excludes the feature values by the number of exclusions from the plurality of feature values on the basis of the distribution distance for each of the plurality of feature values, and selects the one or more input feature values.

7. The information processing system according to claim 6,
wherein the plurality of feature values are N feature values, where N is a positive integer of 2 or more, and
the at least one processor
sets the number of feature values excluded from the N feature values to n, where n is a positive integer,
excludes n feature values from the plurality of feature values on the basis of the distribution distance for each of the N feature values,
performs an evaluation process in which generation of a first tentative prediction model by the machine learning using the training data and calculation of an evaluation index for the first tentative prediction model are executed on the basis of (N-n) feature values,
further excludes n feature values from the (N-n) feature values on the basis of the distribution distance for each of the (N-n) feature values, and
performs an evaluation process in which generation of a second tentative prediction model by the machine learning using the training data and calculation of an evaluation index for the second tentative prediction model are executed on the basis of (N-2n) feature values.

8. The information processing system according to claim 6 or 7, wherein the at least one processor determines, as the number of exclusions, the number at which the highest evaluation index is obtained, or determines, as the number of exclusions, the number at which the lowest evaluation index is obtained.

9. The information processing system according to any one of claims 2 to 4,
wherein the at least one processor
repeats an evaluation process in which generation of a tentative prediction model by the machine learning using the training data and calculation of an evaluation index for the tentative prediction model are executed on the basis of the selected one or more feature values while changing the number of feature values selected from the plurality of feature values on the basis of the distribution distance for each of the plurality of feature values,
determines, on the basis of a plurality of the evaluation indices, the number of feature values selected from the plurality of feature values, as the number of selections, and
selects the feature values by the number of selections from the plurality of feature values as the one or more input feature values on the basis of the distribution distance for each of the plurality of feature values.

10. The information processing system according to any one of claims 6 to 9, wherein the at least one processor executes the evaluation process through cross-validation.

11. The information processing system according to any one of claims 1 to 10, wherein the at least one processor generates the prediction model by executing the machine learning using the one or more input feature values of the training data.

12. The information processing system according to claim 11,
wherein the at least one processor
acquires the one or more input feature values for a molecule of interest whose characteristic value is unknown, and
outputs the characteristic value of the molecule of interest which is obtained by inputting the acquired one or more input feature values to the generated prediction model.

13. The information processing system according to claim 11,
wherein the at least one processor
acquires the one or more input feature values for each of a plurality of molecules of interest whose characteristic value is unknown,
inputs the acquired one or more input feature values to the generated prediction model for each of the plurality of molecules of interest, and acquires the characteristic value of the molecule of interest from the prediction model,
selects at least one molecule of interest among the plurality of molecules of interest on the basis of the characteristic value of each of the plurality of molecules of interest, and
outputs information on the at least one selected molecule of interest.

14. The information processing system according to any one of claims 1 to 13, wherein each of the first molecules and the second molecules is one selected from a nucleic acid, a peptide, a cyclic peptide, a protein, an antibody, and a low-molecular compound.

15. An information processing method executed by an information processing system comprising at least one processor, the method comprising the steps of:
acquiring, for each of a plurality of first molecules, training data representing a plurality of feature values related to the first molecule;
acquiring, for each of a plurality of second molecules, test data representing the plurality of feature values related to the second molecule;
calculating, for each of the plurality of feature values, an index based on a first probability distribution which is a probability distribution in the training data and a second probability distribution which is a probability distribution in the test data; and
selecting, on the basis of the index of each of the plurality of feature values, one or more of the plurality of feature values as one or more input feature values to be used as input parameters of a prediction model for predicting a characteristic value of a molecule on the basis of machine learning.

16. A non-transitory and computer-readable recording medium configured to store an information processing program that allows a computer to execute the steps of:
acquiring, for each of a plurality of first molecules, training data representing a plurality of feature values related to the first molecule;
acquiring, for each of a plurality of second molecules, test data representing the plurality of feature values related to the second molecule;
calculating, for each of the plurality of feature values, an index based on a first probability distribution which is a probability distribution in the training data and a second probability distribution which is a probability distribution in the test data; and
selecting, on the basis of the index of each of the plurality of feature values, one or more of the plurality of feature values as one or more input feature values to be used as input parameters of a prediction model for predicting a characteristic value of a molecule on the basis of machine learning.

17. A method for producing a molecular compound, comprising a generation step of generating a molecular compound having a molecular sequence of the at least one molecule of interest, on the basis of the information of the at least one molecule of interest which is output from the information processing system according to claim 13.
